Europäisches Patentamt

European Patent Office

Office européen des brevets

(19)

(11) **EP 1 280 849 B1**

(12) **EUROPEAN PATENT SPECIFICATION**

(45) Date of publication and mention
of the grant of the patent:
**23.08.2006 Bulletin 2006/34**

(51) Int Cl.:
***C08H 1/00*** *(2006.01)* ***A61K 47/42*** *(2006.01)*

(21) Application number: **01923426.9**

(22) Date of filing: **04.04.2001**

(86) International application number:
**PCT/CA2001/000482**

(87) International publication number:
**WO 2001/074928 (11.10.2001 Gazette 2001/41)**

(54) **PROCESS FOR THE PREPARATION OF PROTEINBASED HYDROGELS**

VERFAHREN ZUR HERSTELLUNG PROTEINHALTIGEN HYDROGELEN

PROCEDE DE PREPARATION D'HYDROGELS A BASE DE PROTEINES

(84) Designated Contracting States:
**AT BE CH CY DE DK ES FI FR GB GR IE IT LI LU MC NL PT SE TR**

(30) Priority: **04.04.2000 US 194412 P**

(43) Date of publication of application:
**05.02.2003 Bulletin 2003/06**

(73) Proprietor: **BIOARTIFICIAL GEL TECHNOLOGIES INC.**
**Montreal,**
**Québec H3A 1L4 (CA)**

(72) Inventors:
• **FAURE, Marie-Pierre**
**Ville St-Laurent, Quebec H4L 1X5 (CA)**
• **PINARD, Karl**
**Montreal, Quebec H2E 2C6 (CA)**
• **BRISSON, Jean-François**
**Montreal, Quebec H1L 3Z8 (CA)**

(74) Representative: **Ahner, Francis et al**
**Cabinet Régimbeau**
**20, rue de Chazelles**
**75847 Paris cedex 17 (FR)**

(56) References cited:
EP-A- 1 025 860    WO-A-95/11924
WO-A-95/15352    US-A- 4 101 380
US-A- 5 122 614    US-A- 5 514 379

• FORTIER G ET AL: "SURFACE MODIFICATION OF HORSERADISH PEROXIDAS4E WITH POLY (ETHYLENE GLYCOL)S OF VARIOUS MOLECULAR MASSES. PREPARATION OF REAGENTS AND CHARACTERIZATION OF HORSERADISH PEROXIDASE-POLY(ETHYLENE GLYCOL) ADDUCTS" BIOTECHNOLOGY AND APPLIED BIOCHEMISTRY,US,ACADEMIC PRESS, vol. 17, 1 February 1993 (1993-02-01), pages 115-130, XP000654783 ISSN: 0885-4513
• D'URSO E M ET AL: "NEW BIOARTIFICIAL POLYMERIC MATERIAL: POLY(ETHYLENE GLYCOL) CROSS-LINKED WITH ALBUMIN. I. SYNTHESIS AND SWELLING PROPERTIES" JOURNAL OF BIOACTIVE AND COMPATIBLE POLYMERS,LANCASTER, PA,US, vol. 9, no. 4, 1 October 1994 (1994-10-01), pages 367-387, XP000606152

**Description**

## FIELD OF THE INVENTION

The present invention relates to a process for the preparation of protein-based hydrogels. More specifically, the present invention is concerned with a process for the preparation in aqueous solution of bioartificial hydrogels having a high water content by cross-linking a protein from a vegetal source (such as hydrolyzed soy protein or hydrolyzed wheat protein), from milk (such as casein) or from a marine source (such as fish protein or algae) with bifunctionalyzed polyethylene glycols. The invention further covers the hydrogels produced by this process and their use in a variety of pharmaceutical, medical and cosmeceutical applications.

## BACKGROUND OF THE INVENTION

Hydrogels are matrices made from polymers of synthetic or natural origin that contain a large quantity of water (usually greater than 50% of their weight). They have the ability to swell and normally do not dissolve in water (1). In general terms, hydrogels are prepared by mixing hydrophilic polymers under conditions that enable physical interactions between the polymers, or the three-dimensional reticulation of these polymers to a degree sufficient to form a gel. Bioartificial or semi-synthetic hydrogels may also be prepared by the covalent addition of bifunctional agents to the proteins so that the proteins are cross-linked to form a three-dimensional matrix.

A wide variety of monomers or polymers have been used in the formation of biomatrixes. The most common are 2-hydroxyethyl methacrylate, referred to as HEMA, poly(ethylene glycol) and poly(ethylene oxide), referred to as PEG and PEO, respectively (2). HEMA, PEG and PEO polymers are advantageous in the preparation of biomatrixes since they are stable under the pH, temperature and salinity conditions commonly encountered in biological media (3).

E. M. D'urso and G. Fortier prepared and characterized hydrogels formed by the combination of bovine serum albumin (BSA) with PEG in alkaline solution (3, 4). In these hydrogels, the protein molecules served as anchor points and were cross-linked between themselves by the use of bifunctionalized PEG. The degree of reticulation was partly dependent on the number of accessible lysyl amino groups available at the proteins' surfaces (6).

The resultant hydrogels were translucid and glassy, and demonstrated mechanical properties in wet conditions very similar to a polyacrylamide gel (4). They also exhibited good BSA retention (0.4-0.5 mg of BSA/mg of PEG), an elevated water content (about 97%) and a good hydrophilicity, making them highly suitable for biomedical applications, particularly in the light of the low immunogenicity associated with proteins modified with PEG (3, 5). Moreover, the high porosity of the hydrogels allowed protein diffusion, suggesting that the hydrogels could be used as controlled delivery systems for macromolecules (3).

U.S. Patent No. 5,514,379 (Weissleder *et al*) teaches the synthesis of biocompatible, biodegradable hydrogels using proteins or polysaccharides with cross-linking agents such as polyvalent derivatives of polyethylene or polyalkylene glycol. More specifically, the cross-linking agents are chosen from among the following group: bis-hydroxysuccinimide ester of polyalkylene glycol ("PAG") diacid, bis-hydroxysulfosuccinimide ester of PAG diacid, bis-imidate of PAG diacid, bis-imidazolide of PAG diacid, bis-imidazolide of PAG, bis-halide of PAG, bis-chloranhydride of PAG diacid, bis (n-amino alkyl) of PAG, and bis (polyoxyalkylene-bis, or bis benzoxazolide of PAG). Reaction between the cross-linking agent and a protein, such as BSA, occurs in DMSO solution. The resultant hydrogels are said to be suitable for loading with diagnostic labels (such as chemotherapeutic drugs, antibiotics, or cells that produce therapeutic agents). Another suggested application is the use of these hydrogels for imaging during interventional procedures of a patient.

U.S. Patent 5,733,563 (Fortier) teaches the preparation in basic solution of bioartificial hydrogels consisting of a three-dimensional cross-linked mixture of a bifunctionalized polyethylene oxide with an albumin-type protein. The bifunctionalized polyethylene oxide is preferably polyethylene glycol and the albumin-type protein is chosen from various sources, such as BSA, lactalbumin and ovalbumin. The patent covers a process for preparing the hydrogels, the hydrogels and various applications for the hydrogels.

The physical characteristics of hydrogels have led to their utilization in a number of ways, including as implant materials (7), contact lenses (8) and wound dressings (9), and as supports for the controlled-release of medications (10).

A major disadvantage encountered with the processes currently available is their slow reaction time, making them inconvenient and/or unsuitable for the mass production of hydrogels. Such processes typically use buffer systems (such as phosphate or borate buffers) to have a controlled cross-linking reaction and to obtain reproducible results. The constant pH that is obtainable through the use of a buffer inhibits the variations that can be observed between different batches of raw materials. However, the use of a buffer also has an impact on the dynamics of the cross-linking reaction, resulting in long reaction times that are impractical for large-scale productions.

There is thus a need for a faster and less expensive process for the preparation of hydrogels having a high-water content which are suitable for a number of pharmaceutical, medical and cosmeceutical applications.

## SUMMARY OF THE INVENTION

It has now been found that the synthesis of hydrogels resulting from the cross-linking of a protein derived from a vegetal source (such as hydrolyzed soy protein or hydrolyzed wheat protein), from milk (such as casein) or from a marine source (such as fish protein or algae) with bis (nitrophenyl carbonate) of PEG conveniently carried out using a strong base in aqueous solution without a buffer system. The reaction time is accelerated significantly and the gels produced have qualities analogous to those produced by the processes known in the art.

Accordingly, the novel hydrogels possess many advantageous properties such as shape retention, shape memory, and high water content (greater than about 95% (w/w) based on the dry weight of the hydrogel). The novel hydrogels also possess good mechanical and optical properties. The hydrogels further possess a plurality of characteristics - biocompatibility, controlled release of medications or drugs, hydrophilic surface, oxygen permeability, and controlled porosity - that should make them highly suitable for pharmaceutical, medical and cosmeceutical applications.

Advantageously, the selection of a protein derived from vegetal sources or milk (such as hydrolyzed soy protein, hydrolyzed wheat protein or casein) to react with bis (nitrophenyl carbonate) of PEG allows the preparation of translucent hydrogels having a high-water content (> 95%) at minimal cost. These proteins are easily obtainable from different sources and therefore are significantly less expensive than animal-based proteins (such as BSA) which have previously been used to make bioartificial hydrogels. Additionally, proteins derived from vegetal sources or milk are free of the prions and viruses that may be present in blood-derived proteins, such as BSA. These features make these proteins highly expedient for the large-scale production of hydrogels.

The present invention therefore provides a process for the preparation of hydrogels, the novel hydrogels produced thereby and a variety of pharmaceutical, medical and cosmeceutical applications based on these novel hydrogels.

The novel hydrogels are polymerized hydrophilic gels essentially consisting of a reticulated mixture of: (a) a bis (nitrophenyl carbonate) of PEG and (b) a protein derived from a vegetal source (such as hydrolyzed soy protein or hydrolyzed wheat protein), from milk (such as casein) or from a marine source (such as fish protein or algae).

Preferably, the protein is hydrolyzed soy protein, hydrolyzed wheat protein or casein,

A variety of pharmaceutical, medical and cosmeceutical applications for the novel hydrogels are also envisaged and methods for preparing the products related to those applications are claimed.

Other objects, advantages and features of the present invention will become apparent upon reading, with reference to the accompanying drawings, the following non-restrictive description of the preferred embodiments thereof.

## BRIEF DESCRIPTION OF THE DRAWINGS

In the appended drawings:

**Figure 1** is a comparison of the reaction kinetics when PEG-soy hydrogels are polymerized in 0.15 N NaOH solution, 0.13 N NaOH solution, 0.12 N NaOH solution and 0.09 N NaOH solution;

**Figure 2** is a comparison of the reaction kinetics for PEG-soy hydrogels synthesized in 0.12 N NaOH solution, 0.09 N NaOH solution and 200 mM borate buffer solution pH 9.4;

**Figure 3** reveals the correspondence between the moment when the solutions begin to solidify and the end-time of polymerization for the synthesis of PEG-soy hydrogels;

**Figure 4** is a graphical comparison of the percentages PEG and hydrolyzed soy protein not incorporated in the resultant hydrogels when the reaction is initiated either in the presence of a strong base or in borate buffer solution;

**Figure 5** is a comparison of the reaction kinetics when PEG-casein hydrogels are polymerized in 0.20 N NaOH solution, 0.19 N NaOH solution and 0.15 N NaOH solution;

**Figure 6** is a comparison of the reaction kinetics when PEG-casein hydrogel is polymerized in 0.19 N NaOH solution, 0.15 N NaOH solution and 200 mM borate buffer solution pH 9.4;

**Figure 7** shows the linear correlation between the initial solidification time and the end-time of polymerization for PEG-casein hydrogels made in NaOH solution;

**Figure 8** is a graphical comparison of the percentages PEG and casein not incorporated in the resultant hydrogels when the reaction is initiated either in the presence of a strong base or in borate buffer solution;

**Figure 9** shows the effect of the ionic strength of the solution on the water content ($C_w$) of the hydrogels produced by the process of the invention;

**Figure 10** shows the effect of the ionic strength of a solution on the water uptake ($C_u$) of the hydrogels produced by the process of the invention;

**Figure 11** shows the effects of the ionic strengths of phosphate buffer solutions on the expansion volumes of the hydrogels produced by the process of the invention;

**Figure 12** shows the effects of the pH of the solution on the water content ($C_w$) of the hydrogels produced by the process of the invention;

**Figure 13** shows the effects of the pH of the solution on the water uptake ($C_u$) of the hydrogels produced by the process of the invention;

**Figure 14** shows the effect of the pH of phosphate and borate buffer solutions on the expansion volumes of the hydrogels produced by the process of the invention; and

**Figure 15** shows the relative uptake of 4-nitrophenol and Methylene Blue by the hydrogels as a function of time.

## DESCRIPTION OF THE PREFERRED EMBODIMENTS

### I. PREPARATION OF PEG-SOY HYDROGEL

#### PEG Activation

In accordance with the invention, PEG was first activated with 4-nitrophenyl chloroformate in order to obtain PEG-dinitrophenyl carbonate (or PEG-(NPC)$^2$), a reagent suitable for reaction with amino groups, in the manner described briefly below.

PEG having a MW of 8kDa was made by Fluka and purchased from Sigma-Aldrich Canada Ltd. (Oakville, Ontario, Canada). 4-Nitrophenyl chloroformate was from Aldrich Chemical Co. (St. Louis, MO, USA) and was used as received. All other chemical and biochemical reagents were of the purest grades available.

PEG-dinitrophenyl carbonates were obtained using the method of Laliberté and Fortier (11). Specifically, prior to use, the PEG was dehydrated by dissolving 1.0 mmole of PEG in acetonitrile and refluxing at 80°C for 24 hours in a Soxhlet™ extractor containing 2.0 g of anhydrous sodium sulfate. The dehydrated solution containing 1.0 mmole of PEG was activated in the presence of at least 10.0 mmoles of 4-nitrophenyl chloroformate in acetonitrile containing up to 5.0 mmoles of triethylamine. The reaction mixture was heated at 60°C for 24 hours. The reaction mixture was cooled and filtered, and the synthesized PEG-dinitrophenyl carbonate was precipitated by adding diethyl ether at room temperature and cooling the solution to 4°C. The percentage of activation was evaluated by following the release of 4-nitrophenol from the activated PEG in 0.1 M borate buffer solution, pH 8.5, at 25°C. The hydrolysis reaction was monitored at 400 nm until a constant absorbency was obtained. The purity was calculated based on the ratio of the amount of 4-nitrophenol released and detected spectrophotometrically against the amount of 4-nitrophenol expected to be released per weight of activated PEG used for the experiment. The purity of the final products was found to be around 100%.

Commercial PEG-dinitrophenyl carbonates suitable for present purposes are available (Sharewater, Huntsville, Alabama, USA).

#### Preparation of Hydrolyzed Soy Protein

Soy protein was purchased from ADM Protein Specialties (Decatur, Illinois).

Hydrolyzed soy protein for reaction with PEG was prepared in the following manner. A 10% (w/v) solution of soy was prepared by combining dry protein with distilled water and homogenizing in a blender. The temperature of the solution obtained was raised to 80°C and 2.15 moles of HCl were added per Kg soy protein. The resultant solution was vigorously agitated for 4 hours at 80°C and allowed to cool to room temperature. The pH of the solution was then increased to between 9 and 10 by adding NaOH while continuing to agitate strongly. The pH of the solution was subsequently lowered to about 4, and the precipitate obtained as a result of the lowering of the pH was collected by centrifugation at 2000 G for 10 minutes. The precipitate containing hydrolyzed soy protein was washed twice by removing the supernatant, mixing with an equivalent volume of distilled water, and centrifuging the solution obtained at 2000 G for 10 minutes. The final precipitate of hydrolyzed soy protein was dissolved in a volume of 1 to 5 mls distilled water per g of protein and the solution equilibrated to pH 7. The neutral solution was lyophilized to obtain a dry powder.

Preparation of Hydrogel Using Activated PEG and Hydrolyzed Soy Protein

To covalently cross-link the activated PEG with hydrolyzed soy protein, the hydrolyzed soy protein was dissolved to a concentration of about 3.5% to about 8% (w/v) in an aqueous solution containing a strong base (such as NaOH, KOH, LiOH, RbOH and CsOH). This solution was combined with an aqueous solution of bifunctionlized PEG having a concentration ranging from about 2% to about 30% (w/v). The resultant solution was vigorously mixed until homogenization occurred. Complete polymerization was observed in times ranging from about 5 minutes to about 120 minutes, depending on the volume of base added.

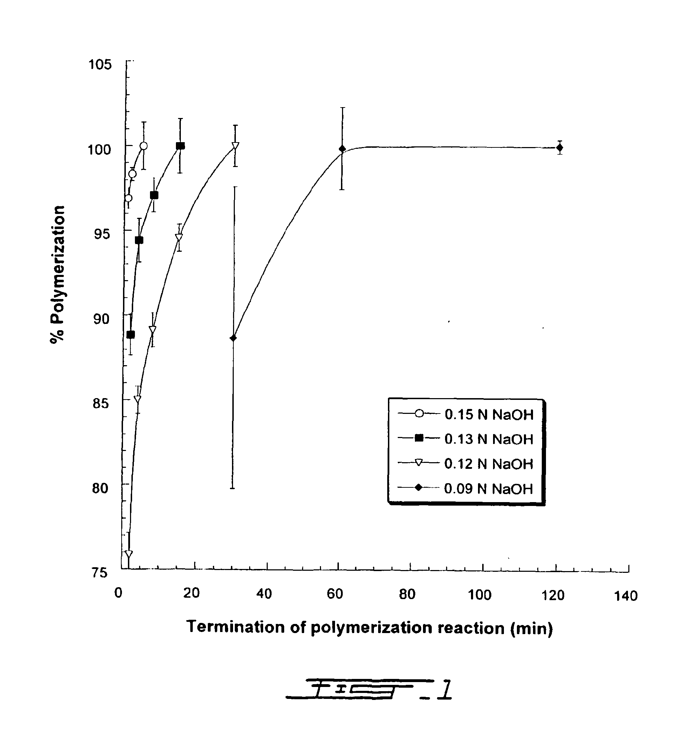

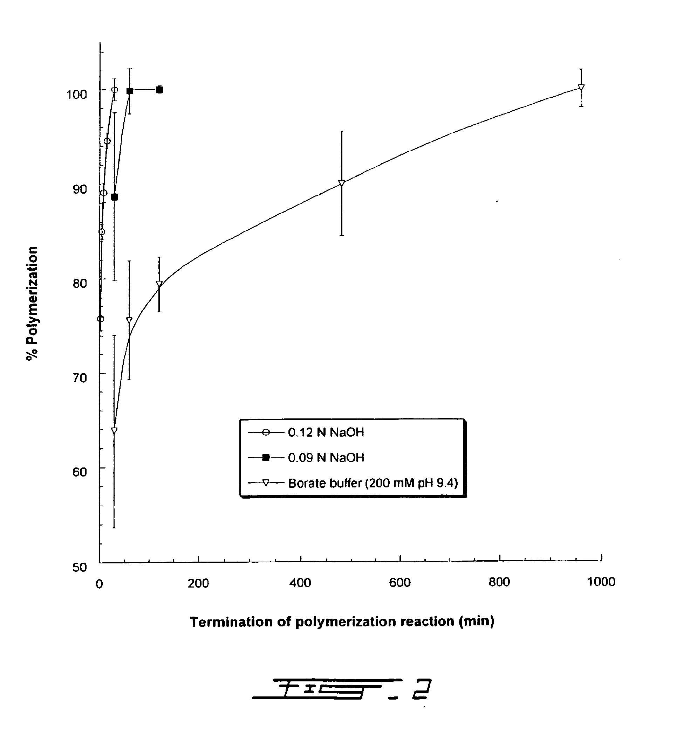

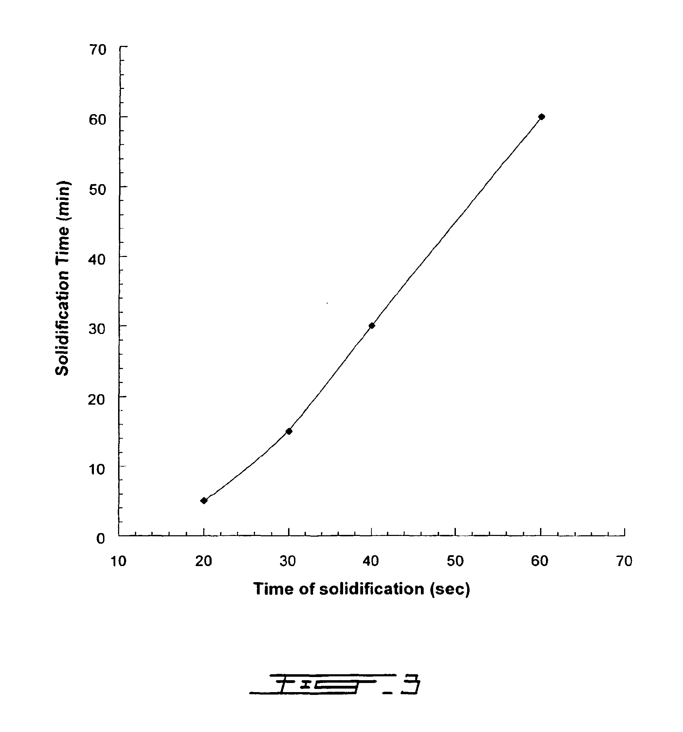

Figure 3 reveals the correspondence between the moments when the solutions begin to solidify and the end-time of polymerization. As revealed in Figures 1 and 2, the speed with which polymerization or cross-linking occurs increases significantly when strong bases are used. It should be noted that the quantity of strong base added to the PEG-soy solution to obtain a given polymerization time will vary depending on the sources of hydrolyzed soy protein and PEG used. Consequently, whenever hydrolyzed soy protein or PEG originating from a different batch or lot is used, it is preferable to conduct preliminary trial experiments to optimize experimental conditions before preparing large quantities of PEG-soy hydrogels.

## II. PREPARATION OF PEG-CASEIN HYDROGEL

PEG Activation

PEG was activated with 4-nitrophenyl chloroformate in order to obtain PEG-dinitrophenyl carbonate, as described above in relation to the PEG-soy hydrogel. (Alternatively, commercially available PEG-dinitrophenyl carbonate could have been used to prepare the hydrogels.)

Preparation of Hydrogel Using Activated PEG and Casein

Casein was purchased from the American Casein Company of Burlington, New Jersey.

To covalently cross-link the activated PEG with casein, the casein was dissolved to a concentration of about 3% to about 9% (w/v) in an aqueous solution containing a strong base (such as NaOH, KOH, LiOH, RbOH and CsOH). This solution was combined with an aqueous solution of bifunctionlized PEG having a concentration ranging from about 3% to about 30% (w/v). The resultant solution was vigorously mixed until homogenization occurred. Complete polymerization was observed in times ranging from about 5 minutes to about 120 minutes, depending on the volume of base added.

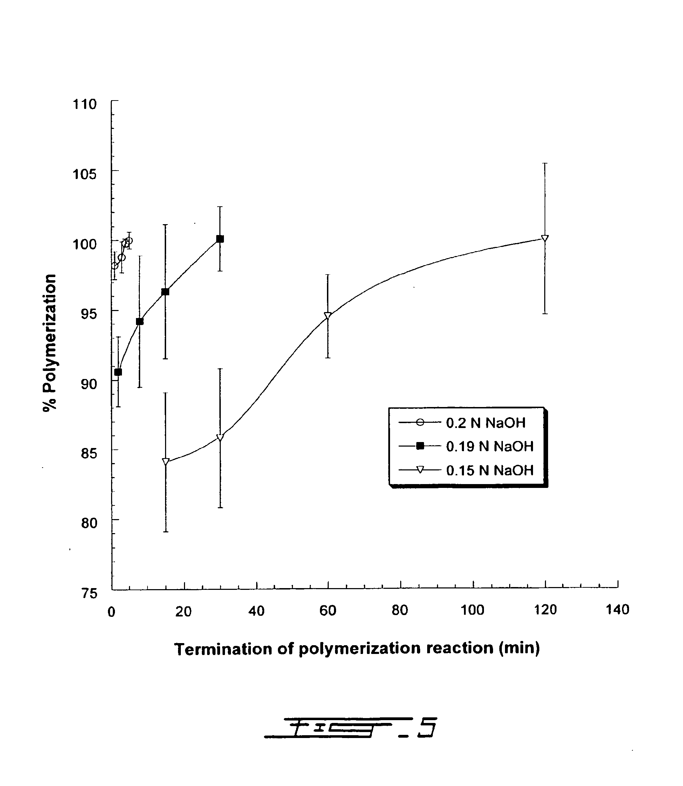

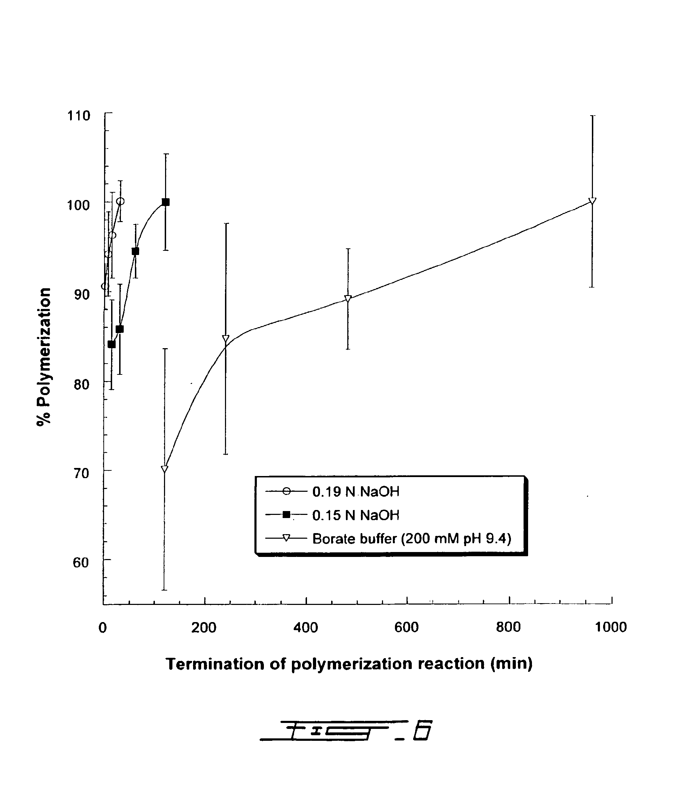

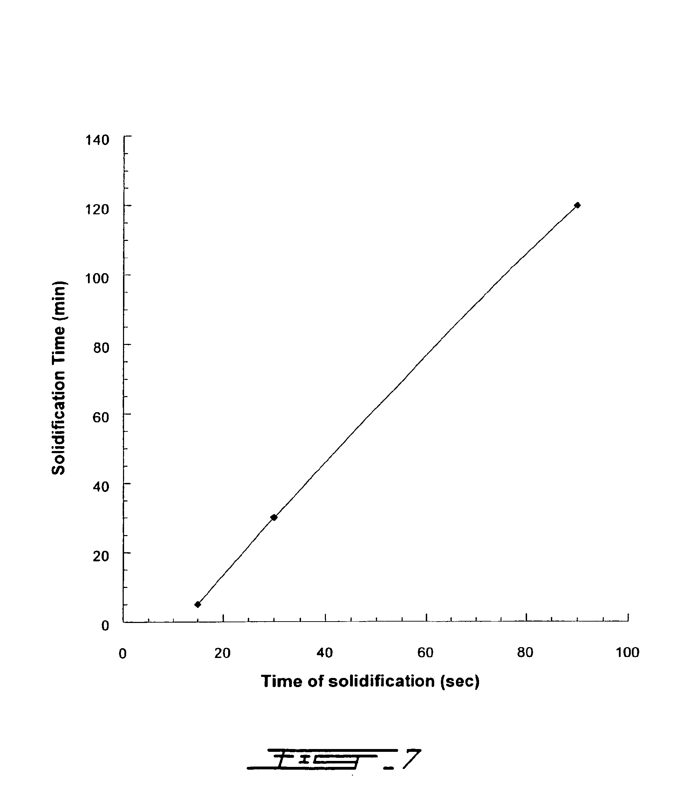

Figure 7 reveals the correspondence between the moment when the solutions begin to solidify and the end-time of polymerization. As revealed in Figures 5 and 6, the speed with which polymerization or cross-linking occurs increases significantly when strong bases are used. It should be noted that the quantity of strong base added to the PEG-casein solution to obtain a given polymerization time will vary depending on the source of casein and PEG used. Consequently, whenever casein or PEG originating from a different batch or lot is used, it is preferable to conduct preliminary trial experiments to optimize experimental conditions before preparing large quantities of PEG-casein hydrogels.

Casein from a number of milks should be suitable for the preparation of hydrogels in accordance with the present method, as would other milk-derived proteins. Indeed, in a test experiment, it was found that complete milk protein does react with bifunctionalized PEG to form an hydrogel, although the resultant gel had weak mechanical properties.Similarly, proteins from a marine source, such as fish protein and algae, would be expected to form hydrogels with similar properties.

## III. PREPARATION OF PEG-WHEAT HYDROGEL

PEG Activation

PEG was activated with 4-nitrophenyl chloroformate in order to obtain PEG-dinitrophenyl carbonate, as described above in relation to the PEG-Soy hydrogel. (Alternatively, commercially available PEG-dinitrophenyl carbonate could have been used to prepare the hydrogels.)

Preparation of Hydrolyzed Wheat Protein

Wheat protein was purchased from ADM Protein Specialties (Decatur, Illinois).

Hydrolyzed wheat protein for reaction with PEG was prepared in the same manner as the hydrolyzed soy protein (described above).

Preparation of Hydrogel Using Activated PEG and Hydrolyzed Wheat Protein

To covalently cross-link the activated PEG with hydrolyzed wheat protein, the protein was dissolved to a concentration of about 8% to about 12% (w/v) in an aqueous solution containing a strong base (such as NaOH, KOH, LiOH, RbOH and CsOH). This solution was combined with an aqueous solution of bifunctionlized PEG having a concentration ranging from about 13% to about 15% (w/v). The resultant solution was vigorously mixed until homogenization occurred. Complete polymerization was observed in times ranging from about 5 minutes to about 120 minutes, depending on the volume of base added.

It should be noted that the quantity of strong base added to the PEG-wheat solution to obtain a given polymerization time will vary depending on the sources of hydrolyzed wheat protein and PEG used. Consequently, whenever wheat protein or PEG originating from a different batch or lot is used, it is preferable to conduct preliminary trial experiments to optimize experimental conditions before preparing large quantities of PEG-wheat hydrogels.

As will be appreciated by one of skill in the art, other vegetal proteins may be substituted for soy or wheat protein in the preparation of hydrogels as described herein without departing from the essence and spirit of the invention. By experimenting with various types of protein, it has been determined that the quantity of carbohydrate attached to the protein may have a direct effect on the suitability of a given source. For example, experiments conducted with pea protein (which has a high carbohydrate content) resulted in hydrogels having weaker mechanical properties.

## EVALUATION OF DEGREE OF WATER CONTENT AND WATER UPTAKE

Under specified solution conditions, the water content ($C_w$) and water uptake ($C_u$) of a synthesized hydrogel can be evaluated by comparing the weight of the dry hydrogel ($W_0$) to the weight of the hydrogel in the swollen state ($W_{sw}$). The water content and water uptake were calculated using the following equations which were extracted from the work of Richard J. LaPorte (14):

$$(1) \qquad C_w = [(W_{sw} - W_0)/W_{sw}] \times 100$$

$$(2) \qquad C_u = [(W_{sw} - W_0)/W_0] \times 100$$

## EXAMPLES

The synthesis of PEG-soy, PEG-casein or PEG-wheat hydrogels using NaOH as a catalyst is characterized by two factors: the solidification time, which corresponds to the time at which the polymerization solution is too viscous to be moulded or formed into a desired shape, and the end-time (or termination time) of polymerisation, which corresponds to the time at which the hydrogel can be washed with minimal loss of PEG and protein.

To evaluate the solidification times relating to a certain amount of NaOH, a simple screening test is used on the different formulations of hydrogels.

### EXAMPLE 1: Preparation and Qualitative Characterization of PEG-Soy Hydrogel Formulations

For hydrogels made of 8 kDa PEG and hydrolyzed soy protein (9% and 6%, respectively), two solutions were prepared: 20 mls of 18 % 8 kDa PEG-(NPC)$^2$ in water and 20 mls of 13.3 % hydrolyzed soy protein in water. The hydrolyzed soy protein solution was divided into 30 samples of 450 $\mu$l, and the samples were adjusted to give a final protein concentration of 12 % by adding 50 $\mu$l of an appropriate volume of NaOH to obtain a NaOH normality ranging from 0.01 N to 0.3 N in the different samples. The PEG-(NPC)$^2$ solution was also separated into 30 samples of 500 $\mu$l. To the different samples of PEG-(NPC)$^2$, 500 $\mu$l of hydrolyzed soy protein at different NaOH normalities were added and mixed. The solution of two polymers was allowed to rest and the solidification time for a specific NaOH normality was noted. When the solidification was greater than 10 minutes, the solution was no longer observed because hydrogels usually do not solidify beyond that time, or have poor mechanical properties if they do.

Two hours following the mixing of the two polymers, the mechanical properties of the hydrogel were qualitatively evaluated using the following scale: very firm, firm, mildly firm, weakly firm and soft.

**Table 1: Data Relating to the Synthesis and Characterization of Different PEG-Soy Hydrogel Formulations**

| NaOH Concentration | Solidification Time | Mechanical properties of the hydrogel (after 2 h) |
|---|---|---|
| 0.01 to 0.03 N | Not measured | Not measured |
| 0.04 to 0.05 N | More then 10 min | (inappropriate) |
| 0.06 N | 4 min 08 sec | Firm (high plasticity and good elasticity) |
| 0.07 N | Not measured | Firm (high plasticity and good elasticity) |
| 0.08 N | 4 min 21 sec | Firm (high plasticity and good elasticity) |
| 0.09 N | 1 min | Very Firm (high plasticity and high elasticity) |
| 0.10 N | 50 sec | Very Firm (high plasticity and high elasticity) |
| 0.11 N | 41 sec | Very Firm (high plasticity and high elasticity) |
| 0.12 N | 38 sec | Very Firm (high plasticity and high elasticity) |
| 0.13 N | 28 sec | Very Firm (high plasticity and high elasticity) |
| 0.14 N | 22 sec | Very Firm (high plasticity and high elasticity) |
| 0.15 N | 20 sec | Very Firm (high plasticity and high elasticity) |
| 0.16 N | Around 20 sec | Very Firm (high plasticity and high elasticity) |
| 0.17 N | Around 20 sec | Very Firm (high plasticity and high elasticity) |
| 0.18 to 0.30 N | Not measured | Not measured |

**EXAMPLE 2: Preparation and Qualitative Characterization of PEG-Casein Hydrogel Formulations**

For hydrogels made of 8 kDa PEG and casein (9% and 7%, respectively), two solutions were prepared: 20 mls of 18 % 8 kDa PEG-$(NPC)^2$ in water and 20 mls of 15.6 % casein in water. The casein solution was divided into 30 samples of 450 $\mu$l, and the samples were adjusted to give a final protein concentration of 14 % by adding 50 $\mu$l of an appropriate volume of NaOH to obtain a NaOH normality ranging from 0.01 N to 0.3 N in the different samples. The PEG-$(NPC)^2$ solution was also separated into 30 samples of 500 $\mu$l. To the different samples of PEG-$(NPC)^2$, 500 $\mu$l of casein at different NaOH normalities were added and mixed. The solution of two polymers was allowed to rest and the solidification time for a specific NaOH normality was noted. When the solidification was greater than 10 minutes, the solution was no longer observed because hydrogels usually do not solidify beyond that time, or have poor mechanical properties if they do.

Two hours following the mixing of the two polymers, the mechanical properties of the hydrogel were qualitatively evaluated using the following scale: very firm, firm, mildly firm, weakly firm and soft.

**Table 2: Data Relating to the Synthesis and Characterization of Different PEG-Casein Hydrogel Formulations**

| NaOH Concentration | Solidification Time | Mechanical properties of the hydrogel (after 2 h) |
|---|---|---|
| 0.01 to 0.06 N | Not measured | Not measured |
| 0.07 to 0.10 N | More then 10 min | (inappropriate) |
| 0.11 N | 1 min 40 sec | Very Firm (high plasticity and high elasticity) |
| 0.12 N | 58 sec | Very Firm (high plasticity and high elasticity) |
| 0.13 N | 37 sec | Very Firm (high plasticity and high elasticity) |

(continued)

| NaOH Concentration | Solidification Time | Mechanical properties of the hydrogel (after 2 h) |
|---|---|---|
| 0.14 N | 41 sec | Very Firm (high plasticity and high elasticity) |
| 0.15 N | 20 sec | Very Firm (high plasticity and high elasticity) |
| 0.16 N | 17 sec | Very Firm (high plasticity and high elasticity) |
| 0.17 N | 11 sec | Very Firm (high plasticity and high elasticity) |
| 0.18 N | 10 sec | Very Firm (high plasticity and high elasticity) |
| 0.19 N | 8 sec | Very Firm (high plasticity and high elasticity) |
| 0.20 N | 10 sec | Very Firm (high plasticity and high elasticity) |
| 0.21 N | 9 sec | Too fast for accurate mixing |
| 0.22 N | 7 sec | Too fast for accurate mixing |
| 0.23 N | 7 sec | Too fast for accurate mixing |
| 0.24 N | 7 sec | Too fast for accurate mixing |
| 0.25 N | 6 sec | Too fast for accurate mixing |
| 0.26 to 0.30 N | Not measured | Not measured |

**EXAMPLE 3: Preparation and Qualitative Characterization of PEG-Wheat Hydrogel Formulations**

For hydrogels made of 8 kDa PEG and hydrolyzed wheat protein (14% and 9%, respectively), two solutions were prepared: 20 mls of 28 % 8 kDa PEG-(NPC)$^2$ in water and 20 mls of 20 % hydrolyzed wheat protein in water. The hydrolyzed wheat protein solution was divided into 30 samples of 450 $\mu$l, and the samples were adjusted to give a final protein concentration of 18 % by adding 50 $\mu$l of an appropriate volume of NaOH to obtain a NaOH normality ranging from 0.01 N to 0.3 N in the different samples. The PEG-(NPC)$^2$ solution was also separated into 30 samples of 500 $\mu$l. To the different samples of PEG-(NPC)$^2$, 500 $\mu$l of hydrolyzed wheat protein at different NaOH normalities were added and mixed. The solution of two polymers was allowed to rest and the solidification time for a specific NaOH normality was noted. When the solidification was greater than 10 minutes, the solution was no longer observed because hydrogels usually do not solidify beyond that time, or have poor mechanical properties if they do.

Two hours following the mixing of the two polymers, the mechanical properties of the hydrogel were qualitatively evaluated using the following scale: very firm, firm, mildly firm, weakly firm and soft.

**Table 3: Data Relating to the Synthesis and Characterization of Different PEG-Wheat Hydrogel Formulations**

| Concentration in NaOH | Time of solidification | Mechanical properties of the hydrogel (after 2 h) |
|---|---|---|
| 0.01 to 0.16 N | Not measured | Not measured |
| 0.17 to 0.18 N | More then 10 min | (inappropriate) |
| 0.19 N | 4 min 15 sec | Firm (high plasticity and good elasticity) |
| 0.20 N | 3 min 13 sec | Firm (high plasticity and good elasticity) |
| 0.21 N | 2 min 55 sec | Firm (high plasticity and good elasticity) |
| 0.22 N | 1 min 35 sec | Firm (high plasticity and good elasticity) |
| 0.23 N | 1 min 22 sec | Firm (high plasticity and good elasticity) |
| 0.24 N | 1 min 49 sec | Firm (high plasticity and good elasticity) |
| 0.25 N | 1 min 25 sec | Mildly Firm (good plasticity and good elasticity) |
| 0.26 N | 2 min 32 sec | Viscous (poor plasticity and poor elasticity) |

(continued)

| Concentration in NaOH | Time of solidification | Mechanical properties of the hydrogel (after 2 h) |
|---|---|---|
| 0.27 N | 1 min 45 sec | Weakly Firm (good plasticity and poor elasticity) |
| 0.28 N | 2 min 35 sec | Viscous (poor plasticity and poor elasticity) |
| 0.29 to 0.30 N | Not measured | Not measured |

### EXAMPLE 4: <u>Measurement of Kinetics and End-times of Polymerization for PEG-Soy Hydrogels</u>

The kinetics and the end-times of polymerization were evaluated through the time-controlled termination of polymerization using a washing step. The end-time of polymerization is defined as the point when the losses in PEG and protein in the washing process reach a minimum. The losses in PEG and protein at the end-time of polymerization also serve to evaluate the quality of the polymerization, as it has been observed that the strength of the mechanical properties of the hydrogels is inversely related to the losses of PEG and protein during the washing steps.

For example, for hydrogels made using a certain batch of 8 kDa PEG and hydrolyzed soy protein (9% and 6% respectively), it was determined that to obtain solidification times of 60 seconds, 40 seconds, 30 seconds and 20 seconds, the polymerization reactions had to occur in 0.09 N, 0.12 N, 0.13 N and 0.15 N NaOH solutions, respectively. The quality and end-times of polymerization of hydrogels synthesized using NaOH were also compared to the hydrogels synthesized in buffer solution by controlled termination of polymerization. In this case, the buffer used to make PEG-soy hydrogels was a 200 mM borate buffer (pH 9.4).

A 100 ml solution of 18 % 8 kDa PEG-(NPC)$^2$ in water was prepared with 20 mls of a 12 % hydrolyzed soy protein solution in 0.09 N NaOH, 20 mls of a 12 % hydrolyzed soy protein solution in 0.12 N NaOH, 20 mls of a 12 % hydrolyzed soy protein solution in 0.13 N NaOH, 20 mls of a 12 % hydrolyzed soy protein solution in 0.15 N NaOH and 20 mls of a 12 % hydrolyzed soy protein solution in 200 mM borate buffer (pH 9.4).

The controlled termination of the polymerization was done in triplicate by transferring 1 ml of PEG solution to three 50-ml tubes. One ml of the desired hydrolyzed soy protein solution (0.09N, 0.12N, 0.13N, 0.15N or 200 mM borate buffer pH 9.4) was added to each tube. Rapidly, the two solutions were mixed and the tube was laid down to allow the hydrogel to form a thin film. The polymerization was stopped at a precise time by pulling the hydrogel from the surface of the tube and by adding 48 ml of 10 mM borate buffer solution (pH 8.5).

The controlled termination of the polymerization was done at intervals of 1 minute, 2 minutes, 3 minutes, 4 minutes and 5 minutes for the polymerizations done in 0.15N NaOH solution. It was done at intervals of 2 minutes, 4 minutes, 8 minutes, 15 minutes and 30 minutes for the polymerizations done in 0.13N and 0.12N NaOH solutions, and at intervals of 8 minutes, 15 minutes, 30 minutes, 60 minutes and 120 minutes for the polymerizations in 0.09N NaOH solution. For hydrogels synthesized in 200 mM borate buffer (pH 9.4), it was done at longer intervals corresponding to 30 minutes, 60 minutes, 120 minutes, 480 minutes and 960 minutes.

The protein and PEG that did not polymerize were recovered in the washing solutions. To ensure that a maximum of protein and of PEG had diffused into the washing solution, the hydrogel was kept under agitation for at least 1 hour during the washing process. To recover all of the unincorporated protein and PEG, three washes were needed. Samples from each wash were withdrawn to determine the quantities of protein and PEG released.

The proteins were measured using the bicinchonic acid assay (BCA) (12). A micro-method kit was prepared in accordance with the manufacturer's directions (Pierce). Two parts of MC reactant (4% cupric sulphate) were mixed with 48 parts of MB reactant (4% BCA in water) and 50 parts of MA reactant (sodium carbonate, sodium bicarbonate and sodium tartrate in 0.2 N NaOH). 100 $\mu$l of this solution were added to 100 $\mu$l of either the protein samples to be analyzed or the control sample. A measurement of the absorbency at 562 nm was made following a 3-hour incubation at 37°C.

The PEG was measured by the formation of a PEG/potassium iodine complex (13). 50 $\mu$l of barium chloride (5% BaCl$_2$ in 1 N HCl) were added to 200 $\mu$l of either the PEG sample to be analyzed or the control. 25 $\mu$l of iodine (2% KI in 0.05N I$_2$) were added to start the reaction. The solution was mixed in the microplates and incubated for 15 minutes in total darkness. After incubation, a measure of the absorbency was made at 532 nm.

To determine the kinetics of the polymerization reaction for the 0.15 N, 0.13 N, 0.12 N and 0.09 N NaOH solutions, the quantity of PEG released during the three washes was subtracted from the amount of PEG present in the initial 2 ml polymerization solution. This was done in order to determine the quantity of PEG present in the final gel, which in turn allowed the determination of the extent (or percentage) of polymerization. One hundred percent polymerization corresponds to the point where the loss of PEG is at its lowest possible value. Figure 1 shows that complete polymerization was attained at intervals of 5 minutes, 15 minutes, 30 minutes and 60 minutes for the 0.15 N, 0.13 N, 0.12 N and 0.09 N NaOH solutions, respectively.

Figure 2 is a comparison of the reaction kinetics when hydrogels were polymerized in 0.12 N NaOH solution, 0.09 N NaOH solution and 200 mM borate buffer solution (pH 9.4). It may be observed that the reaction is significantly longer in borate buffer solution as complete polymerization occurs at 960 minutes in buffer solution compared with 60 minutes for the slowest complete polymerization reaction using NaOH (0.09 N NaOH). This represents a reaction time that is 16 times greater. When compared with reaction using 0.15 N NaOH, reaction in buffer solution is 192 times slower (not shown).

Figure 3 shows the approximately linear correlation between the initial solidification time and the end-time of polymerization for PEG-soy hydrogels made using NaOH.

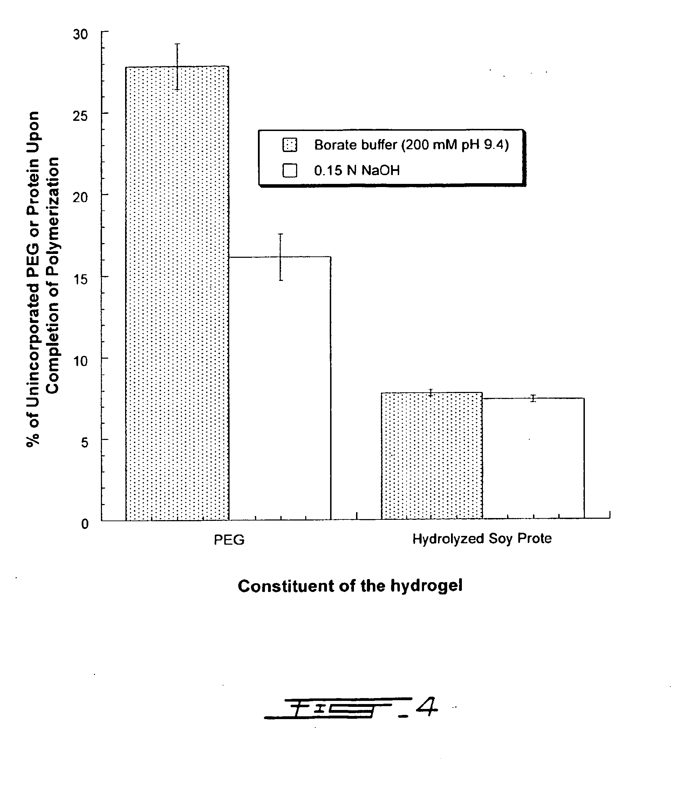

Figure 4 is a bar diagram showing total losses of PEG and hydrolyzed soy protein for hydrogels made in borate buffer and for hydrogels made using NaOH. It may be seen that the losses in PEG are significantly greater when the hydrogels are prepared in borate buffer. A possible explanation for this observation is that the three-dimensional network is not as strong in hydrogels prepared in borate buffer, and this results in hydrogels with weaker mechanical properties (i.e., the degrees of plasticity and elasticity are lower in hydrogels where there is less cross-linking). Figure 4 also shows that the losses in hydrolyzed soy protein do not differ significantly whether the hydrogels are prepared in buffer solution or prepared using NaOH as a catalyst. This may be explained by the fact that the proteins act as anchor points for the bifunctionalized PEG and they can be effectively trapped in the hydrogel even when the reticulation is weak.

## EXAMPLE 5: <u>Measure of Kinetics and End-times of Polymerization for PEG-Casein Hydrogels</u>

The kinetics and the end-times of polymerization were evaluated through the time-controlled termination of polymerization using a washing step. The end-time of polymerization is defined as the point when the losses in PEG and protein in the washing process reach a minimum. The losses in PEG and protein at the end-time of polymerization also serve to evaluate the quality of the polymerization, as it has been observed that the strength of the mechanical properties of the hydrogels is inversely related to the losses of PEG and protein during the washing steps.

For example, for hydrogels made using a certain batch of 8 kDa PEG and casein (9% and 7% respectively), it was determined that to obtain solidification times of 90 seconds, 30 seconds and 15 seconds, the polymerization reactions had to occur in 0.15 N, 0.19 N and 0.20 N NaOH solutions, respectively. The quality and end-times of polymerization of hydrogels synthesized using NaOH were also compared to the hydrogels synthesized in buffer solution by controlled termination of polymerization. In this case, the buffer used to make PEG-Casein hydrogels was a 200 mM borate buffer (pH 9.4).

A 100 ml solution of 18 % 8 kDa PEG-(NPC)[2] in water was prepared with 20 mls of a 14 % casein solution in 0.15 N NaOH, 20 mls of a 14 % casein solution in 0.19 N NaOH, 20 mls of a 14 % casein solution in 0.20 N NaOH and 20 mls of a 14 % casein solution in 200 mM borate buffer (pH 9.4).

The controlled termination of the polymerization was done in triplicate by transferring 1 ml of PEG solution to three 50-ml tubes. One ml of the desired casein solution (0.15N, 0.19N, 0.20N or 200 mM borate buffer pH 9.4) was added to each tube. Rapidly, the two solutions were mixed and the tube was laid down to allow the hydrogel to form a thin film. The polymerization was stopped at a precise time by pulling the hydrogel from the surface of the tube and by adding 48 ml of 10 mM borate buffer solution (pH 8.5).

The controlled termination of the polymerization was done at intervals of 1 minute, 2 minutes, 3 minutes, 4 minutes and 5 minutes for the polymerizations done in 0.20N NaOH solution. It was done at intervals of 2 minutes, 4 minutes, 8 minutes, 15 minutes and 30 minutes for the polymerizations done in 0.19N and at intervals of 8 minutes, 15 minutes, 30 minutes, 60 minutes and 120 minutes for the polymerizations in 0.15N NaOH solution. For hydrogels synthesized in 200 mM borate buffer (pH 9.4), it was done at longer intervals corresponding to 120 minutes, 240 minutes, 480 minutes and 960 minutes.

The protein and PEG which did not polymerize were recovered in the washing solutions. To ensure that a maximum of protein and of PEG had diffused into the washing solution, the hydrogel was kept under agitation for at least 1 hour during the washing process. To recover all of the unincorporated protein and PEG, three washes were needed. Samples from each wash were withdrawn to determine the quantities of protein and PEG released.

The proteins were measured using the bicinchonic acid assay (BCA) (12). A micro-method kit was prepared in accordance with the manufacturer's directions (Pierce). Two parts of MC reactant (4% cupric sulphate) were mixed with 48 parts of MB reactant (4% BCA in water) and 50 parts of MA reactant (sodium carbonate, sodium bicarbonate and sodium tartrate in 0.2 N NaOH). 100 $\mu$l of this solution were added to 100 $\mu$l of either the protein samples to be analyzed or the control sample. A measurement of the absorbency at 562 nm was made following a 3-hour incubation at 37°C.

The PEG was measured by the formation of a PEG/potassium iodine complex (13). 50 $\mu$l of barium chloride (5% $BaCl_2$ in 1N HCl) were added to 200 $\mu$l of either the PEG sample to be analyzed or the control. 25 $\mu$l of iodine (2%

KI in 0.05N I$_2$) were added to start the reaction. The solution was mixed in the microplates and incubated for 15 minutes in total darkness. After incubation, a measurement of the absorbency was made at 532 nm.

To determine the kinetics of the polymerization reaction for the 0.20 N, 0.19 N and 0.15 N NaOH solutions, the quantity of PEG released during the three washes was subtracted from the amount of PEG present in the initial 2 ml polymerization solution. This was done in order to determine the quantity of PEG present in the final gel, which in turn allowed the determination of the extent (or percentage) of polymerization. One hundred percent polymerization corresponds to the point where the loss of PEG is at its lowest possible value. Figure 5 shows that complete polymerization was attained at intervals of 5 minutes, 30 minutes and 120 minutes for the 0.20N, 0.19 N and 0.15N NaOH solutions, respectively.

Figure 6 is a comparison of the reaction kinetics when the hydrogels are polymerized in 0.19 N NaOH solution, 0.15 N NaOH solution and 200 mM borate buffer solution (pH 9.4). It may be observed that the reaction is significantly longer in borate buffer solution as complete polymerization occurs at 960 minutes in buffer solution compared with 120 minutes for the slowest complete polymerization reaction using NaOH (0.15 N NaOH). This represents a reaction time that is 8 times greater. When compared with reaction using 0.20 N NaOH, reaction in buffer solution is 192 times slower (not shown).

Figure 7 shows the linear correlation between the initial solidification time and the end-time of polymerization for PEG-casein hydrogels made using NaOH.

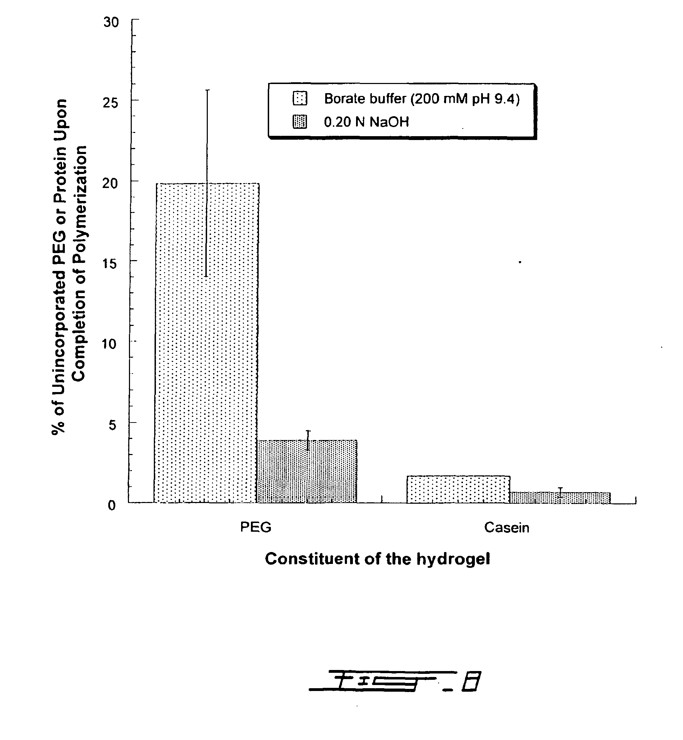

Figure 8 is a bar diagram showing total losses of PEG and casein for hydrogels made in borate buffer and for hydrogels made using NaOH. It may be seen that the losses in PEG are significantly greater when the hydrogels are prepared in borate buffer. A possible explanation for this observation is that the three-dimensional network is not as strong in hydrogels prepared in borate buffer, and this results in hydrogels with weaker mechanical properties (i.e., the degrees of plasticity and elasticity are lower in hydrogels where there is less cross-linking). Figure 8 also shows that the losses in casein are significantly higher when the hydrogels are prepared in buffer solution compared to those prepared using 0.20 N NaOH solution.

**EXAMPLE 6: The Effect of Ionic Strength on the Water Content (C$_w$) and Water Uptake (C$_u$) of PEG-Soy Hydrogels**

Measurements of the water content (C$_w$) and water uptake (C$_u$) were made using solutions having different ionic strengths. Hydrogels made with 8 kDa bifunctionalized PEG and hydrolyzed soy protein (9% and 6 %, respectively) were poured between two plates of glass separated by 1-mm spacers. Hydrogels having a volume of 1.25 mls were subsequently allowed to swell and equilibrate in a solution of 10 mM NaCl to the point where no 4-nitrophenol was detectable by absorbency readings at 400 nm.

Each experiment was performed in triplicate. The hydrogels were allowed to equilibrate in different concentrations of phosphate buffer at pH 6 by washing five times for one hour each time in 40 mls of buffer. The different concentrations of phosphate buffer used were the following: 100 mM, 75 mM, 50 mM, 25 mM, 12.5 mM, 10 mM, 5 mM, 1 mM, 0.1 mM and 0 mM.

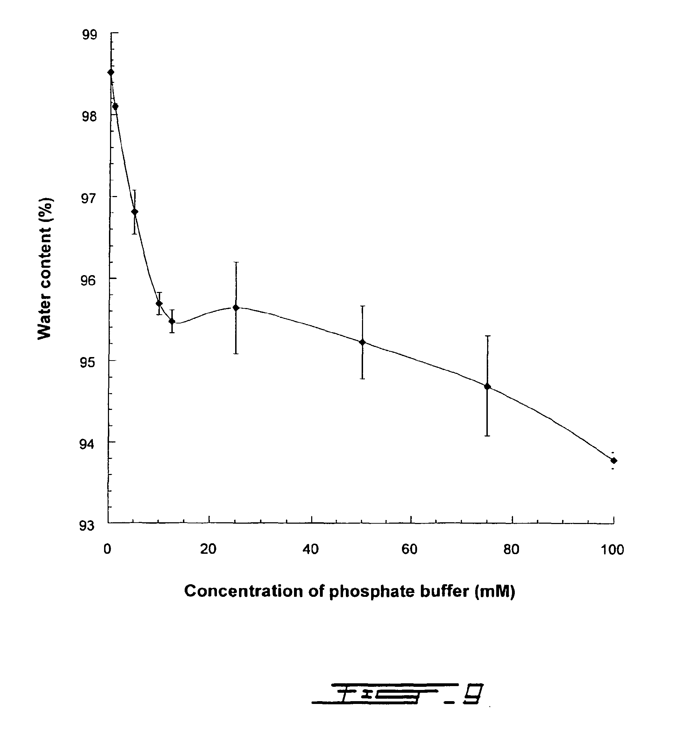

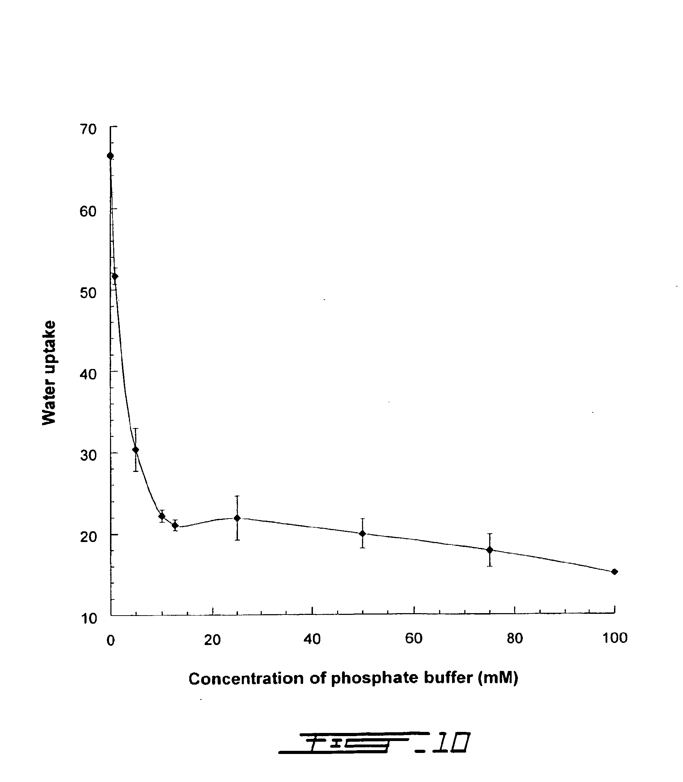

For each concentration of buffer, the hydrogels were removed from solution, the water on their surfaces was blotted and the hydrogels, then in their swollen state ($W_{sw}$), were weighed. The hydrogels were later dried to a constant weight in an oven at 80°C and this dry weight ($W_0$) was measured. The results were then used to calculate the water content ($C_w$) and water uptake ($C_u$) in accordance with equations (1) and (2), above. The effect of the ionic strength of the buffer solutions on the water content and water uptake of the hydrogels is shown graphically in Figures 9 and 10, respectively. It may be observed that the water content ($C_w$) does not differ significantly from about 95% when the buffer concentration ranges between 10 mM and 100 mM. This is even more apparent when the same results are reported in terms of water uptake ($C_u$). It may be seen from Figure 10 that the water uptake is fairly constant with a value of around 20 times the dry weight of the hydrogel when the buffer concentration ranges between 10 mM and 100 mM. There is, however, a dramatic increase in swelling when buffer concentrations lower than 10 mM are used, reaching a maximum when deionized water is used. In the absence of ionic strength, the swelling of the hydrogel can be such as to attain a water content ($C_w$) of about 99 %, corresponding to a water uptake ($C_u$) of about 70 times the dry weight of the hydrogel.

**EXAMPLE 7: The Effect of Ionic Strength on the Expansion Volumes of PEG-Soy Hydrogels**

Measurements of the expansion volumes were made using solutions having different ionic strengths. Hydrogels made with 8 kDa bifunctionalized PEG and hydrolyzed soy protein (9% and 6 %, respectively) were poured between two plates of glass separated by 1-mm spacers. The measurement of the expansion volume was performed in triplicate with a sufficient number of hydrogels having a volume of 1.25 mls. These hydrogels were initially weighed just after synthesis to measure their volumes in their unexpanded state. Subsequently the hydrogels were allowed to equilibrate in different concentrations of phosphate buffer at pH 6 by washing five times for one hour each time in 40 mls of buffer. The different concentrations of phosphate buffer used were the following: 100 mM, 75 mM, 50 mM, 25 mM, 12.5 mM, 10 mM, 5 mM, 1 mM, 0.1 mM and 0 mM.

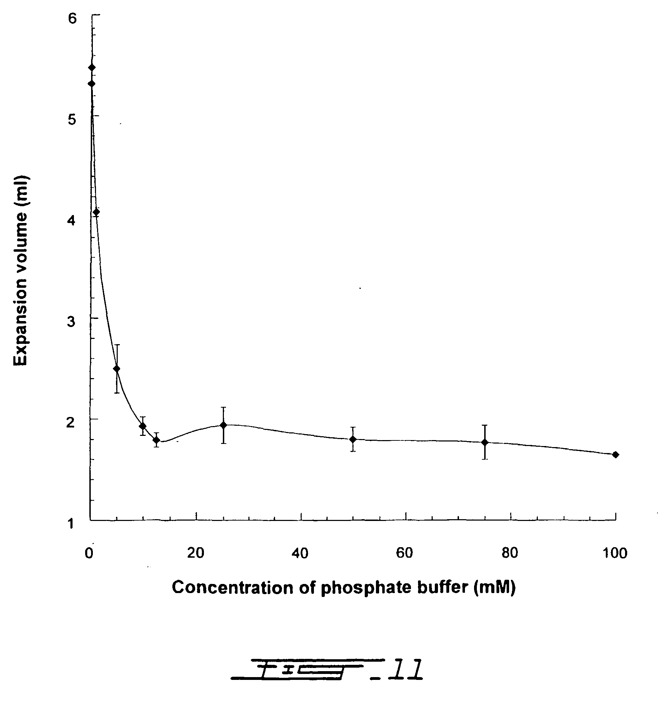

For each concentration of buffer, the hydrogels were removed from solution, the water on their surfaces was blotted and the hydrogels, then in their expanded state, were weighed. The measurement of the expansion volume was made by dividing the weight of the hydrogel in its expanded state by the weight of the hydrogel in its unexpanded state. The effect of the ionic strengths of the buffer solutions on the expansion volumes of the hydrogels is shown graphically in Figure 11. It can be observed that the expansion volume does not differ significantly from 1.8 times the volume of the unexpanded hydrogel when the buffer concentration ranges between 10 mM and 100 mM. There is, however, a dramatic increase in expansion volume when buffer concentrations lower than 10 mM are used, reaching a maximum when deionized water is used. In the absence of ionic strength, the expansion volume of the hydrogel can be such as to attain 5.5 times the volume of the unexpanded hydrogel.

**EXAMPLE 8: The Effect of pH on the Water Content ($C_w$) and Water Uptake ($C_u$) of PEG-Soy Hydrogels**

Measurements of the water content ($C_w$) and water uptake ($C_u$) were made using solutions having different pHs. Hydrogels made with 8 kDa bifunctionalized PEG and hydrolyzed soy protein (9% and 6%, respectively) were poured between two plates of glass separated by 1-mm spacers. Hydrogels having a volume of 1.25 mls were subsequently allowed to swell and equilibrate in a solution of 10 mM NaCl to the point where no 4-nitrophenol was detectable by absorbency readings at 400 nm.

Each experiment was performed in triplicate. The hydrogels were allowed to equilibrate in 10 mM phosphate buffer solution or 10 mM borate buffer solution having different pHs by washing five times for one hour each time in 40 mls of these buffers. Phosphate buffer solutions having pHs of 4, 6 and 7 were used. Borate buffer solutions having pHs of 9 and 11 were used.

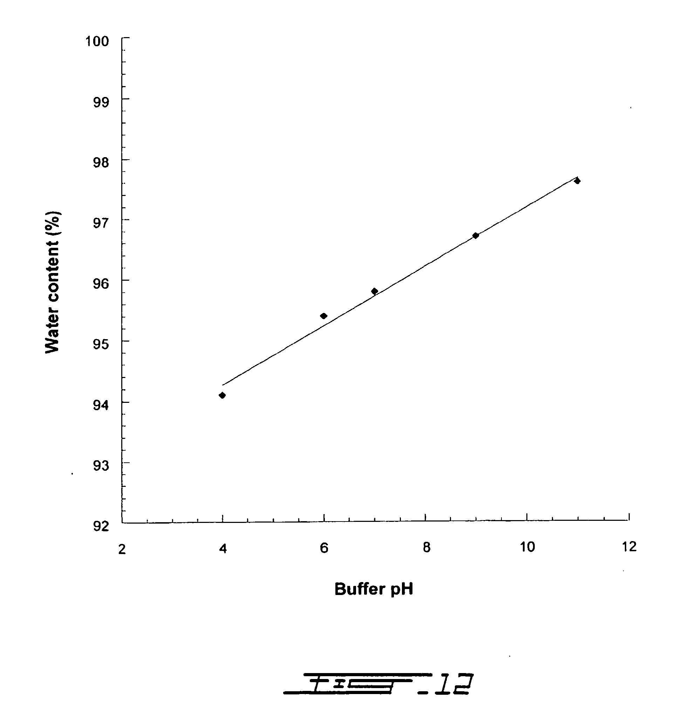

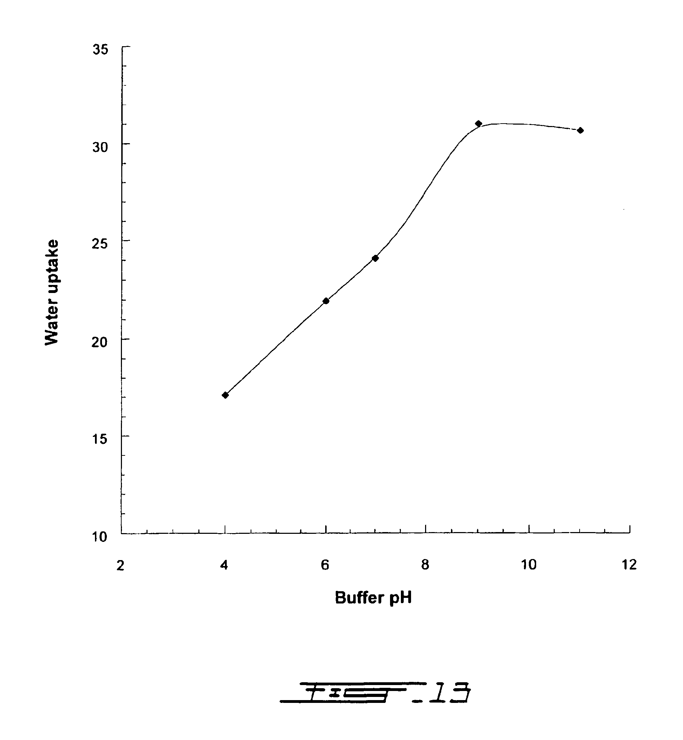

For each concentration of buffer, the hydrogels were removed from solution, the water on their surfaces was blotted and the hydrogels, then in their swollen state ($W_{sw}$), were weighed. The hydrogels were later dried to a constant weight in an oven at 80°C and this dry weight ($W_0$) was measured. The results were then used to calculate the water content ($C_w$) and water uptake ($C_u$) in accordance with equations (1) and (2), above. The effect of the pH of the buffer solutions on the water content and water uptake of the hydrogels is shown graphically in Figures 12 and 13, respectively. It can be observed that the water content ($C_w$) is directly proportional to the pH of the solution, ranging from about 94% to about 97.5% from pH 4 to 11. The same phenomenon can be observed when the water uptake ($C_u$) is considered. It may be seen from Figure 13 that the water uptake is directly proportional to the pH of the solution, ranging from about 17 times the dry weight to about 30 time the dry weight from pH 4 to 11. These variations in water content ($C_w$) and water uptake ($C_u$) can be explained by the low solubility of the hydrolyzed soy protein comprising the hydrogel at low pH and its increased solubility at high pH.

**EXAMPLE 9: The Effect of pH on the Expansion Volumes of PEG-Soy Hydrogels**

Measurements of the expansion volumes were made using solutions having different pHs. Hydrogels made with 8 kDa bifunctionalized PEG and hydrolyzed soy protein (9% and 6%, respectively) were poured between two plates of glass separated by 1-mm spacers. The measurement of the expansion volume was performed in triplicate with a sufficient number of hydrogels having a volume of 1.25 mls. These hydrogels were initially weighed just after synthesis to measure their volumes in their unexpanded state. Subsequently the hydrogels were allowed to equilibrate in 10 mM phosphate buffer solution or 10 mM borate buffer solution having different pHs by washing five times for one hour each time in 40 mls of these buffers. Phosphate buffer solutions having pHs of 4, 6 and 7 were used. Borate buffer solutions having pHs of 9 and 11 were used.

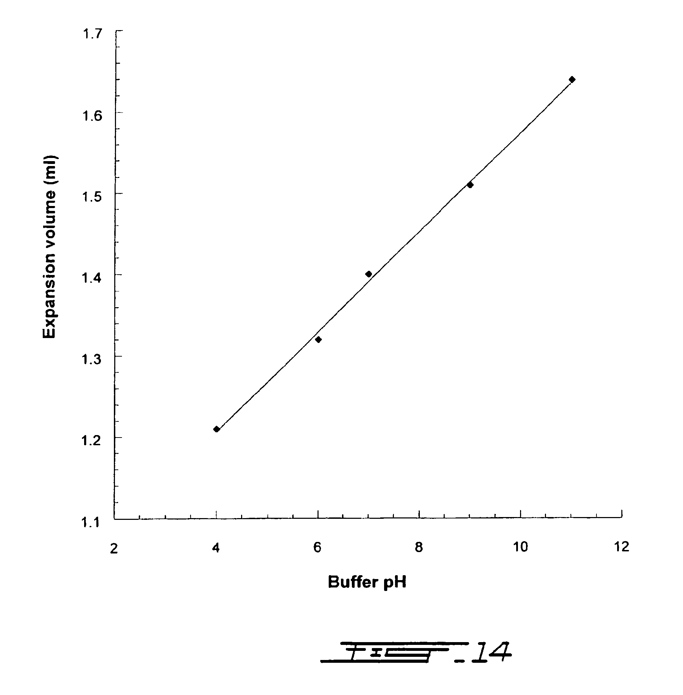

For each concentration of buffer, the hydrogels were removed from solution, the water on their surfaces was blotted and the hydrogels, then in their expanded state, were weighed. The measurement of the expansion volume was made by dividing the weight of the hydrogel in its expanded state by the weight of the unexpanded hydrogel. The effect of the pH of the buffer solutions on the expansion volumes of the hydrogels is shown graphically in Figure 14. It can be observed that the expansion volume is directly proportional to the pH of the solution, ranging from about 1.2 times the unexpanded volume of the hydrogel to about 1.65 times the unexpanded volume of the hydrogel from pH 4 to 11. These variations in expansion volume can be explained by the low solubility of the hydrolyzed soy protein comprising the hydrogel at low pH and its increased solubility at high pH.

**EXAMPLE 10: Measurements of the Uptake of Two Different Models of Active Ingredients in PEG-Soy Hydrogels**

Measurements of the uptake times of actives ingredients in sample PEG-Soy hydrogels were made using 4-itrophenol and Methylene Blue as model molecules. Hydrogel films were made with 8 kDa bifunctionalized PEG and hydrolyzed soy protein (9% and 6%, respectively) by pouring the polymeric solution between two plates of glass separated by 1-mm spacers. Hydrogels were subsequently cut into small squares and allowed to swell and equilibrate in a 10 mM phosphate buffer solution pH 6 to the point where no 4-nitrophenol was detectable by absorbency readings at 400 nm. In their swollen state, the volume of those hydrogels was 745 $\mu$l $\pm$ 22 $\mu$l.

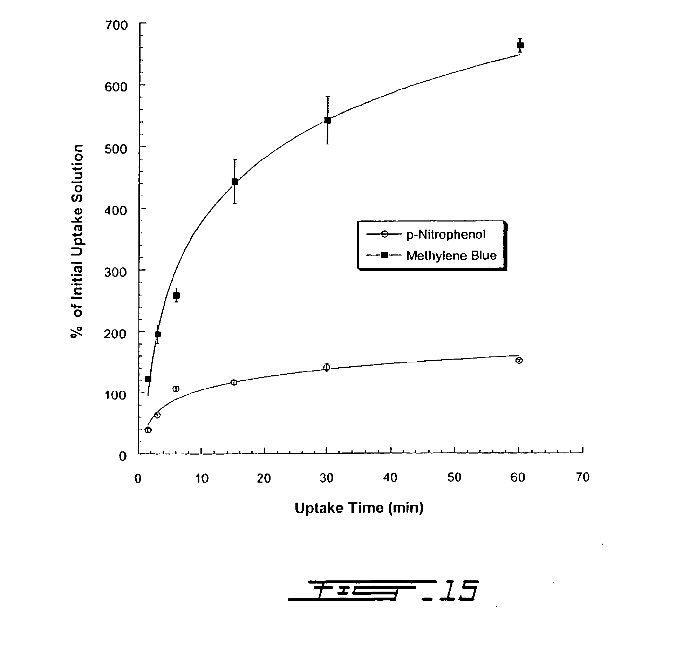

The evaluation of the uptake time of 4-nitrophenol and Methylene Blue was made by first preparing a 1 ppm uptake solution of Methylene Blue and a 0.4 % solution of 4-nitrophenol. Three hydrogels were place in contact with a fresh 90-ml volume of one these uptake solutions for the following time intervals: 1½ min, 3 min, 6 min, 15 min, 30 min and 60 min before their were removed from solution. After their removal, the hydrogels were carefully blotted of excess solution and were each transferred into 30 mls of 10 mM of phosphate buffer solution pH 6. The hydrogels were allowed to equilibrate in the solution by agitating for 24 hours to ensure that the equilibrium state was reached. The uptake of 4-nitrophenol and Methylene Blue was assumed to correspond to the amount of those model molecules released in the washing solution. The 4-nitrophenol in solution was measured through absorbency readings at 400 nm by comparing the results to a standard curve ranging from 80 to 1 $\mu$g/ml. Methylene Blue was measured through absorbency readings at 655 nm by comparing the results to a standard curve ranging from 3 to 0.0025 ppm. To evaluate the relative uptake of either of these model molecules, the total quantity of molecules in the 30-ml solution was taken to correspond to the initial volume of the hydrogel (745 $\mu$l). The concentration of the model molecules reported in the hydrogel was then compared (in percentage) to the initial concentration of the uptake solution. Figure 15 shows the relative uptake of 4-nitrophenol and Methylene Blue as a function of time. It may be seen that both molecules diffused very rapidly into the hydrogel and reached the same concentration as the uptake solution, in less than 1½ minutes in the case of the Methylene Blue and in about 15 minute in the case of the 4-Nitrophenol. Methylene Blue even became concentrated in the hydrogel, to the point where it reached a concentration corresponding to more than 600 % that of the surrounding solution. This phenomenon may be caused by the latent charge of the hydrogel or the natural affinity of Methylene Blue for protein. As lots of other active ingredients or medicaments have affinities to protein similar to that demonstrated here by Methylene Blue, it may be assumed that the same type of concentration of active ingredients would be observed with them.

Experimental conditions may be modified, as known to those of skill in the art, in order to achieve hydrogels having consistencies suitable for different applications. Thus, for example, the plasticity and/or elasticity of the hydrogels may be modified by varying the amounts of PEG and protein used to synthesize the hydrogels, or the nature of the protein used.

Further, the physical appearance of the hydrogels may be adjusted depending on the application. For example, the hydrogel solution may be synthesized in different forms (such as films, blocks, etc.) by pouring it between glass plates or in a plastic mould. Once set, the hydrogel may be broken up to form particles of different sizes. Those particles could also be made by suspension or emulsion polymerization.

Applications for the novel hydrogels prepared by the process described herein include the following:

- Drug-release devices that could be used in systemic, intra-tumoral, sub-cutaneous, topical, transdermic and rectal applications;
- Wound dressings or artificial skins;
- Solid humidified reaction mediums for diagnostic kits, for use in fundamental research (PCR, RT-PCR, *in situ* hybridization, *in situ* labelling with antibodies or other markers such as peptides, DNA or RNA probes, medicaments or hormones, etc.), etc.;
- Transport mediums for cells, tissues, organs, eggs, organisms, etc.;
- Tissue culture mediums, with or without active agents, for fundamental research, or commercial and therapeutic applications;
- Electrode materials (with or without enzymes);
- Iontophoretic membranes;
- Protective humidified mediums for tissue sections (such as replacement coverglasses for microscope slides); and
- Matrices for the immobilization of enzymes or proteins for *in vivo, in vitro* or *ex vivo* use as therapeutic agents, bioreactors or biosensors.
- Cosmeceutical applications, such as skin hydrators or moisturizers A dry form of these hydrogels (obtained after dehydration under vacuum or in acetone) can also be used in the following manner: firstly, as a water or exudate absorbent in wound dressing and secondly, as a slow or controlled drug release device.

## LIST OF REFERENCES

1. N. A. Peppas, *Hydrogels in Medicine and Pharmacy,* Vol. 1-3, CRC Press, Boca Raton, FL, 1987.

2. Corkhill, P. H., C. J. Hamilton and B. J. Tighe. 1990. *Crit. Rev. Biocompat.,* 5:363; Graham, N. B. 1992. *Poly*

*(ethylene glycol) Chemistry: Biotechnical and Biomedical Applications,* J. Milton Harris, ed., New York: Plenum Press, p. 263.

3. D'Urso, E. M. and G. Fortier. 1994. *J. Bioactive and Compatible Polymers,* 9:368.

4. D'Urso, E. M. and G. Fortier. 1994. *Biotechnology Techniques,* 8:71.

5. Abuchowski, A., J. R. McCoy, T. van Es, N. C. Palczuk and F. F. Davis. 1977. J. Biol. Chem., 252: 3582.

6. E. M. D'Urso, J.-F. Jacques and G. Fortier, *Bioapplication of Poly(ethylene glycol)-Albumin Hydrogels: Matrix for Enzyme Immobilization* in <u>Hydrogels and Biodegradable Polymers for Bioapplications</u> (1996: American Chemical Society), Chapter 4, p. 27.

7. Bray, J. C. and E. W. Merrill. 1973. J. *Biomed. Mater. Res.,* 7:431; Bekhuis, W. H. and B. E. McCarey. 1985. *Invest. Ophthalmol. Vis. Sci.*, 26:1634.

8. Schaefer, D. and R. Schaefer. 1985. To Alcon Laboratories Inc., U.S. Patent 4,546,123 A; Donshik, P. *et al.* 1988. *CLAO J.,* 14:191; Gruber, E. 1988. *CLAO J.,* 14:195.

9. Wang, P. J. and N. A. Samji. 1981. *Polym. Sci. Technol.*, 14:29; Nalbandian, R. M. et al. 1987. *J. Biomed. Mater. Res.,* 21: 1135.

10. L. Olanoff and J. M. Anderson. 1979. *J. Pharm. Sci.,* 68:1151; Law, T. K., A. T. Florence and T. L. Whateley. 1986. *Int. J. Pharm.,* 33:65; Graham, N. B. and M. E. McNeil. 1988. *Makromol. Chem., Macromol. Symp.,* 19:255.

11. M. Laliberté and G. Fortier. Surface Modification of Horseradish Peroxidase with Poly(ethylene glycol) of various molecular masses. I: Preparation of Reagents and Characterization of Horseradish Peroxidase-Poly(ethylene glycol) Adducts. 1993. *Biotechnology and Applied Biochemistry,* 17:115-130.

12. R.E. Brown, K.L. Jarvis and K.J. Hyland. Protein Measurement using Bicinchonic Acid: Elimination of Interfering Substance. 1989. *Analytical Biochemistry,* 180: 136-139.

13. G.E.C. Sim and T.J. Sharpe. A Method for Estimation of Polyethylene Glycol in Plasma Protein Fraction. *Analytical Biochemistry*,107:60-63.

14. Richard J. LaPorte, Hydrophilic Polymer Coatings for Medical Devices: Structure/Properties, Development, Manufacture and Applications. 1997. Technomic Publishing Company, Inc., Lancaster, Pennsylvania, USA, at pages 41-44.

**Claims**

1. A process for preparing a polymerized hydrophilic water-swellable hydrogel comprising:

   (a) providing an aqueous solution containing a bis (nitrophenyl carbonate) of PEG;
   (b) providing a solution of a protein derived from a vegetable source, marine source or milk in a non-buffered aqueous solution under basic conditions that are provided by a strong base, selected from the group consisting of LiOH, NaOH, KOH, RbOH and CsOH,
   (c) reacting the solutions of (a) and (b) in order to induce polymerization.

2. A process as defined in claim 1, wherein said protein is selected from the group consisting of hydrolyzed soy protein, hydrolyzed wheat protein, pea protein and casein.

3. A process as defined in any of claims 1 or 2, wherein said strong base is NaOH or KOH.

4. A polymerized hydrophilic water-swellable hydrogel produced by a process as defined in any one of claims 1 to 3.

5. A polymerized hydrophilic water-swellable hydrogel comprising a protein selected from a vegetal source, marine source or milk crosslinked with bis (nitrophenyl carbonate) of PEG
wherein the crosslinking reaction is conducted in an aqueous solution under basic conditions provided by a strong base without a buffer system,
and wherein said strong base is selected from the group consisting of LiOH, NaOH, KOH, RbOH, and CsOH.

6. A hydrogel as defined in claim 5, wherein said protein is selected from the group consisting of hydrolyzed soy protein, hydrolyzed wheat protein, pea protein and casein.

7. Use of a hydrogel as defined in any one of claims 4 to 6 in the making of a medicament comprising a physiologically active compound to be administered to a mammal in a therapeutically effective amount.

8. The use of claim 7, wherein said medicament is administered together with a physiologically acceptable carrier.

9. The use of claim 7 or 8, wherein said hydrogel consists of a controlled drug release composition in a physiological form suitable for systemic, intra-tumoral, sub-cutaneous, topical, transdermic or rectal administration.

10. The use of any one of claims 7 to 9, wherein said hydrogel constitutes a wound dressing or artificial skin.

11. Use of a hydrogel as defined in any one of claims 4 to 6 as a matrix for the immobilization of enzymes or proteins.

12. The use of claim 11, wherein said matrix serves as a therapeutic agent, or is part of a bioreactor or a biosensor.

13. Use of a hydrogel as defined in any one of claims 4 to 6 as a solid humidified reaction medium.

14. Use of a hydrogel as defined in any one of claims 4 to 6 as a transport medium.

15. Use of a hydrogel as defined in any one of claims 4 to 6 as a tissue culture medium.

16. Use of a hydrogel as defined in any one of claims 4 to 6 as electrode material.

17. Use of a hydrogel as defined in any one of claims 4 to 6 as an iontophoretic membrane.

18. Use of a hydrogel as defined in any one of claims 4 to 6 as a protective humidified medium.

**Patentansprüche**

1. Verfahren zur Herstellung eines polymerisierten hydrophilen, in Wasser quellbaren Hydrogels, umfassend:

   (a) Bereitstellen einer wässrigen Lösung, die ein Bis(nitrophenyl-carbonat) von PEG enthält;
   (b) Bereitstellen einer Lösung eines Proteins, das aus einer pflanzlichen Quelle, einer marinen Quelle oder Milch abstammt, in einer nicht gepufferten wässrigen Lösung unter basischen Bedingungen, die von einer starken Base bereitgestellt werden, welche ausgewählt ist aus der Gruppe bestehend aus LiOH, NaOH, KOH, RbOH und CsOH,
   (c) Umsetzen der Lösungen von (a) und (b), um die Polymerisation zu induzieren.

2. Verfahren nach Anspruch 1, in dem das Protein ausgewählt ist aus der Gruppe bestehend aus hydrolysiertem Sojaprotein, hydrolysiertem Weizenprotein, Erbsenprotein und Casein.

3. Verfahren nach irgendeinem der Ansprüche 1 oder 2, in dem die starke Base NaOH oder KOH ist.

4. Polymerisiertes hydrophiles, in Wasser quellbares Hydrogel, hergestellt durch ein Verfahren, wie in irgendeinem der Ansprüche 1 bis 3 definiert.

5. Polymerisiertes hydrophiles, in Wasser quellbares Hydrogel, umfassend ein Protein, das aus einer pflanzlichen Quelle, einer marinen Quelle oder Milch ausgewählt ist und mit Bis(nitrophenylcarbonat) von PEG vernetzt ist, wobei die Vernetzungsreaktion in einer wässrigen Lösung unter basischen Bedingungen durchgeführt wird, die von einer starken Base ohne ein Puffersystem bereitgestellt werden,

und wobei die starke Base ausgewählt ist aus der Gruppe bestehend aus LiOH, NaOH, KOH, RbOH und CsOH.

6. Hydrogel nach Anspruch 5, in dem das Protein ausgewählt ist aus der Gruppe bestehend aus hydrolysiertem Sojaprotein, hydrolysiertem Weizenprotein, Erbsenprotein und Casein.

7. Verwendung eines Hydrogels nach irgendeinem der Ansprüche 4 bis 6 bei der Herstellung eines Medikaments, das eine physiologisch aktive, einem Säuger in einer therapeutisch wirksamen Menge zu verabreichende Verbindung umfasst.

8. Verwendung nach Anspruch 7, bei der das Medikament zusammen mit einem physiologisch annehmbaren Träger verabreicht wird.

9. Verwendung nach Anspruch 7 oder 8, bei der das Hydrogel aus einer Zusammensetzung zur gesteuerten Arzneistofffreisetzung in einer physiologischen Form besteht, die für eine systemische, intratumorale, subkutane, topische, transdermale oder rektale Verabreichung geeignet ist.

10. Verwendung nach irgendeinem der Ansprüche 7 bis 9, bei der das Hydrogel einen Wundverband oder künstliche Haut darstellt.

11. Verwendung eines Hydrogels nach irgendeinem der Ansprüche 4 bis 6 als Matrix für die Immobilisierung von Enzymen oder Proteinen.

12. Verwendung nach Anspruch 11, bei der die Matrix als therapeutisches Mittel dient oder Teil eines Bioreaktors oder eines Biosensors ist.

13. Verwendung eines Hydrogels nach irgendeinem der Ansprüche 4 bis 6 als festes befeuchtetes Reaktionsmedium.

14. Verwendung eines Hydrogels nach irgendeinem der Ansprüche 4 bis 6 als Transportmedium.

15. Verwendung eines Hydrogels nach irgendeinem der Ansprüche 4 bis 6 als Gewebekulturmedium.

16. Verwendung eines Hydrogels nach irgendeinem der Ansprüche 4 bis 6 als Elektrodenmaterial.

17. Verwendung eines Hydrogels nach irgendeinem der Ansprüche 4 bis 6 als Iontophoresemembran.

18. Verwendung eines Hydrogels nach irgendeinem der Ansprüche 4 bis 6 als schützendes befeuchtetes Medium.

**Revendications**

1. Procédé pour la préparation d'un hydrogel polymérisé hydrophile apte à gonfler dans l'eau, comprenant :

   (a) la production d'une solution aqueuse contenant un bis(carbonate de nitrophényle) de PEG ;
   (b) la production d'une solution d'une protéine provenant d'une source végétale, d'une source marine ou de lait, dans une solution aqueuse non tamponnée, dans des conditions basiques qui sont produites par une base forte choisie dans le groupe consistant en LiOH, NaOH, KOH, RbOH et CsOH,
   (c) la mise en réaction des solutions de (a) et (b) afin d'induire une polymérisation.

2. Procédé tel que défini dans la revendication 1, dans lequel ladite protéine est choisie dans le groupe consistant en la protéine de soja hydrolysée, la protéine de blé hydrolysée, la protéine de pois et la caséine.

3. Procédé tel que défini dans la revendication 1 ou 2, dans lequel ladite base forte est NaOH ou KOH.

4. Hydrogel polymérisé hydrophile apte à gonfler dans l'eau, produit par un procédé tel que défini dans l'une quelconque des revendications 1 à 3.

5. Hydrogel polymérisé hydrophile apte à gonfler dans l'eau, comprenant une protéine choisie parmi une protéine d'origine végétale, d'origine marine ou de lait, réticulée avec du bis(carbonate de nitrophényle) de PEG,

dans lequel la réaction de réticulation est effectuée dans une solution aqueuse, dans des conditions basiques produites par une base forte sans un système tampon,
et dans lequel ladite base forte est choisie dans le groupe consistant en LiOH, NaOH, KOH, RbOH et CsOH.

6. Hydrogel tel que défini dans la revendication 5, dans lequel ladite protéine est choisie dans le groupe consistant en la protéine de soja hydrolysée, la protéine de blé hydrolysée, la protéine de pois et la caséine.

7. Utilisation d'un hydrogel tel que défini dans l'une quelconque des revendications 4 à 6, dans la fabrication d'un médicament comprenant un composé physiologiquement actif à administrer en une quantité thérapeutiquement efficace à un mammifère.

8. Utilisation selon la revendication 7, dans laquelle ledit médicament est administré conjointement avec un véhicule physiologiquement acceptable.

9. Utilisation selon la revendication 7 ou 8, dans laquelle l'hydrogel consiste en une composition libérant une substance active, sous une forme physiologique appropriée à l'administration systémique, intra-tumorale, sous-cutanée, topique, transdermique ou rectale.

10. Utilisation selon l'une quelconque des revendications 7 à 9, dans laquelle ledit hydrogel constitue un pansement ou de la peau artificielle.

11. Utilisation d'un hydrogel tel que défini dans l'une quelconque des revendications 4 à 6, en tant que matrice pour l'immobilisation d'enzymes ou de protéines.

12. Utilisation selon la revendication 11, dans laquelle ladite matrice sert d'agent thérapeutique, ou fait partie d'un bioréacteur ou d'un biocapteur.

13. Utilisation d'un hydrogel tel que défini dans l'une quelconque des revendications 4 à 6, en tant que milieu réactionnel solide humidifié.

14. Utilisation d'un hydrogel tel que défini dans l'une quelconque des revendications 4 à 6, en tant que milieu de transport.

15. Utilisation d'un hydrogel tel que défini dans l'une quelconque des revendications 4 à 6, en tant que milieu pour culture de tissu.

16. Utilisation d'un hydrogel tel que défini dans l'une quelconque des revendications 4 à 6, en tant que matériau d'électrode.

17. Utilisation d'un hydrogel tel que défini dans l'une quelconque des revendications 4 à 6, en tant que membrane iontophorétique.

18. Utilisation d'un hydrogel tel que défini dans l'une quelconque des revendications 4 à 6, en tant que milieu protecteur humidifié.

FIG. 1

**Termination of polymerization reaction (min)**

FIG. 2

FIG. 3

_Fig_4

**Termination of polymerization reaction (min)**

_FIG_ _5_

**Termination of polymerization reaction (min)**

_FIG_ 6

FIG.7

FIG. 8

Concentration of phosphate buffer (mM)

Concentration of phosphate buffer (mM)

FIG. 10

FIG. 11

FIG. 12

_FIG. 13_

_Fig. 14_